(19) Europäisches Patentamt
European Patent Office
Office européen des brevets

(11) EP 1 879 027 B1

(12) **EUROPEAN PATENT SPECIFICATION**

(45) Date of publication and mention of the grant of the patent:
**12.06.2013 Bulletin 2013/24**

(21) Application number: **06732431.9**

(22) Date of filing: **27.04.2006**

(51) Int Cl.:
***G01N 33/68*** *(2006.01)* ***G01N 33/94*** *(2006.01)*

(86) International application number:
**PCT/JP2006/308880**

(87) International publication number:
**WO 2006/118212 (09.11.2006 Gazette 2006/45)**

(54) **AGENT FOR PREVENTING AND TREATING PANCREATITIS**

MITTEL ZUR VORBEUGUNG UND BEHANDLUNG EINER PANKREATITIS

AGENT DE PRÉVENTION ET DE TRAITEMENT DES PANCRÉATITES

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.04.2005 JP 2005130408**

(43) Date of publication of application:
**16.01.2008 Bulletin 2008/03**

(73) Proprietor: **CytoPathfinder, Inc.**
**Tokyo**
**103-0022 (JP)**

(72) Inventors:
- **YAMAGUCHI, Isamu**
  **6660129 (JP)**
- **HAMADA, Kentaro,**
  **503, Gracia Confort 21,**
  **Kyoto-shi,**
  **Kyoto 6078221 (JP)**
- **KASHIHARA, Yasunari**
  **Yokohama-shi,**
  **Kanagawa 2230051 (JP)**

(74) Representative: **Jacobi, Markus Alexander et al**
**Isenbruck Bösl Hörschler LLP**
**Eastsite One**
**Seckenheimer Landstrasse 4**
**68163 Mannheim (DE)**

(56) References cited:
- **KOLLER ELIZABETH A ET AL: "Pancreatitis associated with atypical antipsychotics: From the Food and Drug Administration's MedWatch surveillance system and published reports." PHARMACOTHERAPY, vol. 23, no. 9, September 2003 (2003-09), pages 1123-1130, XP008098936 ISSN: 0277-0008**
- **VARGA GABOR ET AL: "Effect of deramciclane, a new 5-HT receptor antagonist, on cholecystokinin-induced changes in rat gastrointestinal function" EUROPEAN JOURNAL OF PHARMACOLOGY, vol. 367, no. 2-3, 19 February 1999 (1999-02-19), pages 315-323, XP002505195 ISSN: 0014-2999**
- **ASAI FUMITOSHI ET AL: "Effects of R-102444, a prodrug 5-HT2 receptor antagonist, in rat and mouse models of acute and chronic pancreatitis" JAPANESE JOURNAL OF PHARMACOLOGY, vol. 88, no. Supplement 1, 2002, page 277P, XP008098908 & 75TH ANNUAL MEETING OF THE JAPANESE PHARMACOLOGICAL SOCIETY; KUMAMOTO, JAPAN; MARCH 13-15, 2002 ISSN: 0021-5198**
- **OGAWA TAKETOSHI ET AL: "Pharmacological profiles of R-96544, the active form of a novel 5-HT2A receptor antagonist R-102444." EUROPEAN JOURNAL OF PHARMACOLOGY, vol. 457, no. 2-3, 20 December 2002 (2002-12-20), pages 107-114, XP002505196 ISSN: 0014-2999**
- **LI J P ET AL: "ROLES OF 5-HT RECEPTORS IN THE RELEASE AND ACTION OF SECRETIN ON PANCREATIC SECRETION IN RATS" AMERICAN JOURNAL OF PHYSIOLOGY, AMERICAN PHYSIOLOGICAL SOCIETY, BETHESDA, MD, US, vol. 280, no. 4, PART 01, 1 April 2001 (2001-04-01), pages G595-G602, XP008033772 ISSN: 0002-9513**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 879 027 B1

- BONHAUS D W ET AL: "THE PHARMACOLOGY AND DISTRIBUTION OF HUMAN 5-HYDROXYTRYPTAMINE2B (5-HT2B) RECEPTOR GENE PRODUCTS: COMPARISON WITH 5-HT2A AND 5-HT2C RECEPTORS" BRITISH JOURNAL OF PHARMACOLOGY, BASINGSTOKE, HANTS, vol. 1155, 1 January 1995 (1995-01-01), pages 622-628, XP009039172 ISSN: 0007-1188
- YOSHINO T. ET AL: 'Possible involvement of 5-HT2 receptor activation in aggravation of diet-induced acute pancreatitis in mice' J. PHARMACOL. EXP. THER. vol. 283, no. 3, 1997, pages 1495 - 1502, XP002936527
- MIDORIKAWA T.: 'The effect of ketanserin on acute pancreatic injury models in the rat' THE JOURNAL OF JAPAN PANCREAS SOCIETY vol. 9, 1994, pages 443 - 451, XP003005611
- OGAWA T. ET AL.: 'Effects of R-102444 and its active metabolite R-96544, selective 5-HT2A receptor antagonists, on experimental acute and chronic pancreatitis: Additional evidence for possible involvement of 5-HT2A receptors in the development of experimental pancreatitis' EUR. J. PHARMACOL. vol. 521, 22 September 2005, pages 156 - 163, XP005107591
- NOMURA S. ET AL.: '5-HT2A receptor antagonist increases circulating adiponectin in patients with type 2 diabetes' BLOOD COAGUL. FIBRINOLYSIS vol. 16, 2005, pages 423 - 428, XP003005612
- OGUCHI H. ET AL.: 'Effects of the S2-serotonergic receptor antagonist, ketanserin, on cerulein-induced pancreatitis in the rat' LIFE SCI. vol. 50, no. 10, 1992, pages 733 - 737, XP003005613
- BOURIN MICHEL ET AL: "5-HT2 receptors and anxiety", DRUG DEVELOPMENT RESEARCH, vol. 65, no. 3, July 2005 (2005-07), pages 133-140, ISSN: 0272-4391

## Description

### FIELD OF THE INVENTION

The present invention relates to a pharmaceutical agent for prophylaxis or treatment of pancreatitis.

### BACKGROUND OF THE INVENTION

Pancreatitis is an inflammation of the pancreas, which includes acute pancreatitis and chronic pancreatitis. Pancreatic juice contains digestive enzymes such as amylase (hydrolyzes carbohydrates), trypsin (hydrolyzes proteins), and lipase (hydrolyzes fats). Pancreatitis is a condition in which these enzymes cause autodigestion of the pancreas because the pancreatic juice does not flow smoothly owing to alcohol abuse, gallstones, or the like. Pancreatitis is classified into two major types, a mild type in which interstitial edema and peripancreatic fat necrosis are observed, and a severe type in which extensive peripancreatic and intrapancreatic fat necrosis, pancreatic parenchymal necrosis, and hemorrhage are observed. When an inflammation occurs in the pancreas, amylase and lipase contained in the pancreatic juice are released into the blood and urine. Therefore, it is possible to diagnose pancreatitis by measuring the levels of amylase and lipase in the blood and urine.

As a therapy of acute pancreatitis, the following are commonly used at present. When the pancreatitis is mild, secretion of the enzymes is suppressed by not taking food and drink. When the digestive enzymes are released into the blood, complications such as respiratory failure and renal failure may sometimes occur. Therefore, in treatment settings in Japan, pancreatic enzyme inhibitors such as aprotinin preparation, gabexate mesilate (FOY), and camostat mesilate are administered. Most of mild and moderate cases of pancreatitis are alleviated by such therapies within about one week. In severe cases of pancreatitis, since damages develop in various organs, systemic management is required in an intensive care unit. When a patient falls into the state of shock, a large amount of adrenal cortex hormone preparation as an antiinflammatory drug is administered together with an aprotinin preparation. With respect to mechanism of action of therapeutic agents for pancreatitis that has been known so far, no relation to serotonin has been suggested.

Serotonin (5-hydroxytryptamine: 5-HT) is one of neurotransmitters. Serotonin is contained most in the brain and the gastrointestinal tracts, and besides them, abundantly in platelets. It is considered that 5-HT released from the gastrointestinal tracts into blood is taken up into platelets. When platelets are activated, 5-HT is released. Serotonin is an autacoid. In other words, serotonin is a chemical compound that is produced by cells of a certain type, affects functions of cells of different types on the same site, and plays a role as a local hormone or a transmitter.

Currently, seven kinds (14 subtypes) of serotonin receptors having distinct actions and localizations have been known:

5-HT1A, 5-HT1B, 5-HT1D, 5-HT1E, 5-HT1F
5-HT2A, 5-HT2B, 5-HT2C
5-HT3, 5-HT4, 5-HT5A, 5-HT5B, 5-HT6, 5-HT7

Among them, 5-HT2 receptor is classified into subtypes, A, B, and C and has the following distribution and actions.

5-HT2A is present in vascular smooth muscle, platelets, the lungs, the central nerve, and the gastrointestinal tracts and involved in blood vessel contraction, platelet coagulation, and release of serotonin from platelets.

5-HT2B is present mainly in the peripheries and also in the brain and involved in muscle contraction of the stomach fundus of rat. It is also involved in relaxation of blood vessels.

5-HT2C is present in the central nerve and also in the peripheries and involved in eating function and automatism. It is also involved in relaxation of blood vessels.

Various 5-HT2 receptor antagonists have been found so far and developed as therapeutic agents for migraine, inhibitors for platelet coagulation, therapeutic agents for mental disorders such as schizophrenia, anxiety disorder, and alcoholism, and the like. However, it has not been known that an antagonist of the 5-HT2A receptor is effective for treatment of pancreatitis.

Japanese Patent Application Laid-Open Publication No. 1998-212232 discloses a therapeutic agent for pancreatitis containing a certain kind of diarylalkane derivative as an effective component and also shows that this compound group has a 5-HT2 receptor antagonistic activity. However, there is no suggestion that this activity is based on a 5-HT2A receptor antagonistic activity.

Japanese Patent Application Laid-Open Publication No. 2000-63270 discloses that a prophylactic and therapeutic agent for pancreatitis containing Sarpogrelate hydrochloride ((±)-1-[o-[2-(m-methoxyphenyl)ethyl]phenoxy)-3-(dimethylamino)-2-propyl hydrogen succinate hydrochloride) as an effective component. However, there is no suggestion that this activity is based on the 5-HT2A receptor antagonistic activity.

Further, Yoshino, et al., (J. Pharmacol. Exp. Ther. 283(3), 1495-1502, 1997) suggest that the activation of the 5-HT2 receptor is involved in the onset of acute pancreatitis but do not mention at all the relation between the antagonistic activity on the 5-HT2A, 5-HT2B, and 5-HT2C receptors and pancreatitis.

The references cited in the specification are listed below. The entire contents of these documents are incorporated herein by reference. It is not intended that the contents of any one of these documents be regarded as prior art to the present invention.

Japanese Patent Application Laid-Open Publication No. 1998-212232
Japanese Patent Application Laid-Open Publication No. 1998-147528
Yoshino, et al., J. Pharmacol. Exp. Ther. 283(3), 1495-1502, 1997

Document Koller et al, Pharmacotherapy 2003; 23(9): 1123 - 1130, describes a pharmacovigilance study which investigates the relative numbers and clinical chraracteristics of pancreatitis in patients treated with the atypical antipsychotic agents clozapine, olanzapine, and risperidone, versus the conventional neuroleptic, haloperidol.

Document Oguchi et al, Life Sciences, vol. 50, pp. 733-737, describes the effects of the $S_2$-serotonergic receptor antagonists, ketaserin on cerulin-induced pancreatitis in the rat.

Document Bonhaus et al., Br.J.Pharmacology (1005), 115, 622-628, describes and compares the pharmacology and distribution of the gene products of the 5-HT2 receptor subtyps, 5-HT2a, 5HT-2b and 5HT-2C. Affinities (pki values and binding activity) for different antagonists, such as spiperone, ketanserin and ritanserin are disclosed.

## SUMMARY OF THE INVENTION

The present invention aims to provide a pharmaceutical agent for prophylaxis or treatment of pancreatitis.

The present inventors found that among various 5-HT2 receptors, particularly the 5-HT2A receptor is involved in the onset of pancreatitis. In other words, the present invention provides a method of identifying candidate substances for prevention and treatment of pancreatitis comprising determining whether a test substance has a 5-HT2A receptor antagonistic activity. Preferably, the method of identifying candidate substances for the prophylactic and therapeutic agent for pancreatitis of the present invention comprises determining the binding activities (pKis) of a test substance to the 5-HT2A and 5-HT2B receptors and identifying the test substance as a candidate substance for the prophylactic and therapeutic agent for pancreatitis when the binding activity to the 5-HT2A receptor is higher at least by 1.0 than the binding activity to the 5-HT2B receptor. Still more preferably, the method comprises determining the binding activity to the 5-HT2C receptor and identifying the test substance as a candidate substance for the prophylactic and therapeutic agent for pancreatitis when the binding activity to the 5-HT2A receptor is higher at least by 1.0 than the binding activity to the 5-HT2B receptor and the 5-HT2C receptor.

The binding activity (pKi) of a receptor antagonist to a receptor is represented by the ability of the receptor antagonist to competitively inhibit the binding between the receptor and a ligand. A ligand is a substance that is known to bind to its corresponding receptor, and for example, DOI is known as a ligand of the 5-HT2A receptor. Binding activity (pKi) can be determined by a method known in the art. Specifically, a dissociation constant (Kd) of binding between a ligand labeled with a radioactive material or the like and a receptor is determined. Next, the binding between the labeled ligand and the receptor is determined in the presence of a receptor antagonist at various concentrations. When the concentration of the receptor antagonist that inhibits 50% of the receptor binding is expressed as IC50 and the concentration of the labeled ligand as L, an inhibition constant (Ki) can be obtained by the following formula:

$$Ki = IC50/(1+L/Kd)$$

Binding activity (pKi) is pKi=-logKi.

The present invention provides a pharmaceutical composition for prophylaxis and treatment of pancreatitis having the 5-HT2A receptor antagonist as an effective component, where the binding activity (pKi) of the 5-HT2A receptor antagonist to the 5-HT2A receptor is higher at least by 1.0 than the binding activity to the 5-HT2B receptor and is higher at least by 1.0 than the binding activity to the 5-HT2C receptor and wherein the 5-HT2A receptor antagonist is selected from the group consisting of risperidone, ziprasidone, paliperidone, iloperidone, quetiapine, nefazodone, perospirone, aripiprazole, zotepine, mirtazapine, sertindole, asenapine, blonanserin, spiperone, amperozide, and chlorpromazine. Note that the 5-HT2A receptor antagonist is not ketanserin, sarpogrelate hydrochloride, or a compound having the following formula:

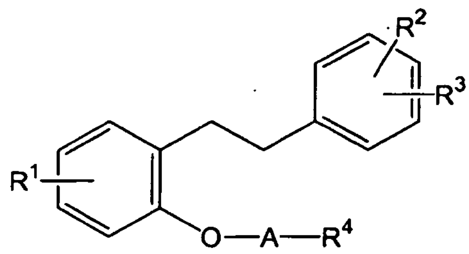

wherein $R^1$ represents a hydrogen or halogen atom, $R^2$ and $R^3$ are the same or different and represent a hydrogen or halogen atom, or a $C_1$-$C_4$ alkoxy group, $R^4$ represents a 5- or 6-membered cyclic amino group that may be substituted and may contain an oxygen or sulfur atom, wherein the substituent represents a $C_1$-$C_{20}$ aliphatic acyloxy group that may contain a hydroxyl group or double bond when present on the carbon atom, and represents a $C_1$-$C_4$ alkyl group when present on the nitrogen atom, and A represents a $C_1$-$C_4$ alkylene group.

Preferably, in the pharmaceutical composition of the present invention, the binding activity (pKi) of the 5-HT2A receptor antagonist to the 5-HT2A receptor is 7.0 or higher, more preferably 8.0 or higher, and still more preferably 9.0 or higher. Further, in the pharmaceutical composition of the present invention, the binding activity (pKi) of the 5-HT2A receptor antagonist to the 5-HT2B receptor is preferably 6.0 or lower. More preferably, in the pharmaceutical composition of the present invention, the binding activity (pKi) of the 5-HT2A receptor antagonist to the 5-HT2C receptor is 7.0 or lower.

In the pharmaceutical composition of the present invention, the 5-HT2A receptor antagonist is selected from the group consisting of psychotropic agents such as risperidone, ziprasidone, paliperidone, iloperidone, quetiapine, nefazodone, perosipirone, aripiprazole, zotepine, mirtazapine, sertindole, asenapine, blonanserin, spiperone, amperozide, and chlorpromazine

BRIEF DESCRIPTION OF THE DRAWINGS

Fig. 1 represents effects of a 5-HT2A antagonist, a 5-HT2B antagonist, and a 5-HT2C antagonist on amylase activity and lipase activity in plasma of mouse with cerulein-induced pancreatitis;

Fig. 2 represents effects of various 5-HT2A antagonists on the amylase activity and the lipase activity in the plasma of mouse with cerulein-induced pancreatitis; and

Fig. 3 represents effects of 5-HT2B antagonists on the amylase activity and the lipase activity in the plasma of mouse with cerulein-induced pancreatitis. DETAILED

DESCRIPTION OF THE PREFERRED EMBODIMENTS

As a 5-HT2A receptor antagonist used as an effective component of a pharmaceutical composition of the present invention, a substance can be used which inhibits the functions of a 5-HT2A receptor, including, psychotropic agents such as risperidone, ziprasidone, paliperidone, iloperidone, quetiapine, nefazodone, perospirone, aripiprazole, zotepine, mirtazapine, sertindole, asenapine, blonanserin, spiperone, amperozide, and chlorpromazine, . Further examples of 5-HT2A receptor antagonists are AMI-193, MDL11939, ZD-3638 (Zeneca Group plc), ICI-169369 (Zeneca Group plc), amperozide (Pharmacia & Upjohn AB), Mk-212 (Merck & Co. Inc.), S-16924 (Servier), deramciclane (EGIS Gyogyszergyar RT), TY-11223 (Toa Eiyo KK), S-21357-1 (Servier), S-14956 (Servier), S-17828 (Servier), RP-71602 (Rhone-Poulenc SA), LY-367265 (Eli Lilly & Co), LY-433221 (Eli Lilly & Co), SEP-89406 (Sepracor Inc), FG-5938 (Kabi Pharmacia AB), FG-5974 (Pharmacia & Upjohn Inc), QF-2004B (Universidade de Santiago de Compostela), JL-13 (Therabel Research SA), S-14297 (Servier), LEK-8829 (Lek Pharmaceutical and Chemical Co), R-107500 (Janssen Pharmaceutica NV), S-35120 (Servier), IT-657 (Bristol-Myers Squibb Pharma Co), F-97013-GD (FAES Farma SA), nantenine (Tohoku University), gamma-mangostin (Tohoku University)), abaperidone (Ferrer Internacional SA), EMR-62218 (Merck KGaA), SL-65.0472 (Sanofi-Synthelabo), ACP-103 (ACADIA Pharmaceuticals Inc), AC-90179 (ACADIA Pharmaceuticals Inc), NRA-0045 (Taisho Pharmaceutical Co Ltd), Y-39241 (Mitsubishi Pharma Corp), ST-2329 (Sigma-Tau Ind Farm Riunite SpA), S(+)-mirtazapine (NV Organon), SM-13496 (Sumitomo Pharmaceuticl Co., Ltd.), eplivanserin (Sanofi-Synthelabo), SUN-C5147 (Suntory Ltd), E-2101 (Eisai Co., Ltd.), 100907 (Aventis Pharmaceuticals Inc), flibanserin (Boehringer Ingerlheim Corp), and the like. These substances are commercially available or can be easily prepared by those skilled in the art based on their known structures.

Further, the following compounds (Glennon et al., Curr Top Med Chem, 2002, 2, 539-558) are the 5-HT2A receptor antagonists:

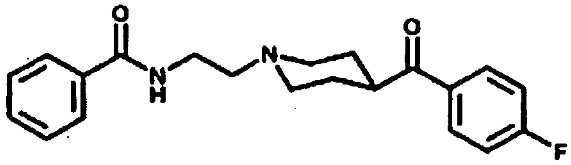

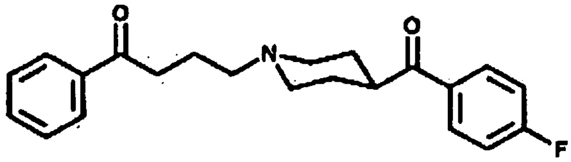

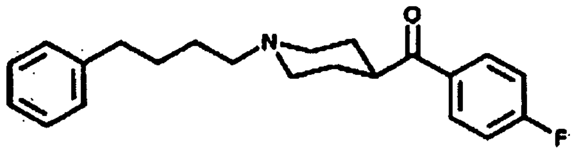

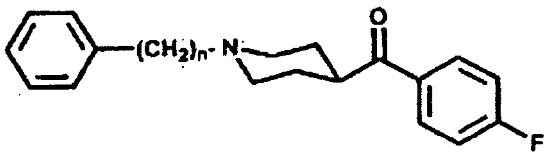

[n=1, 2, 3, or 5]

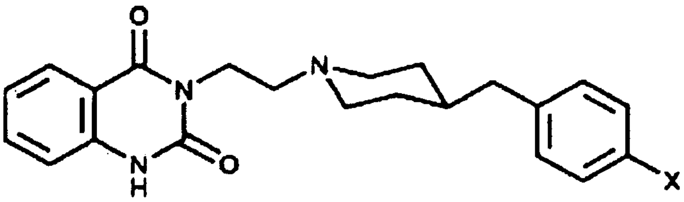

[X=F or H]

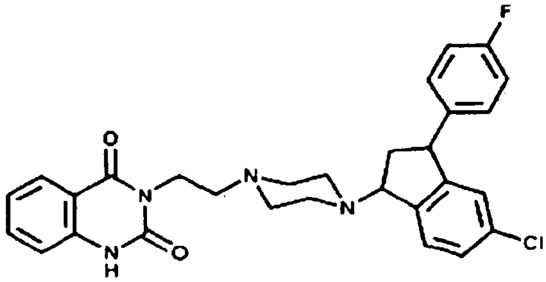

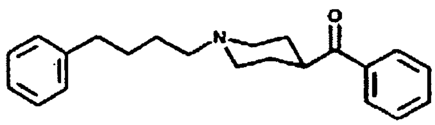

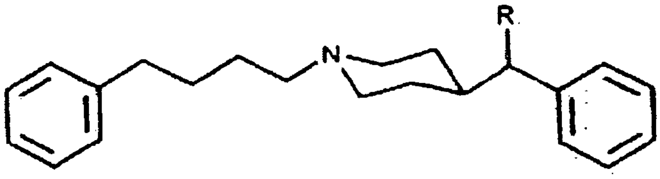

[R=H, Ph, $CH_2Ph$, or $CH_2CH_2Ph$]

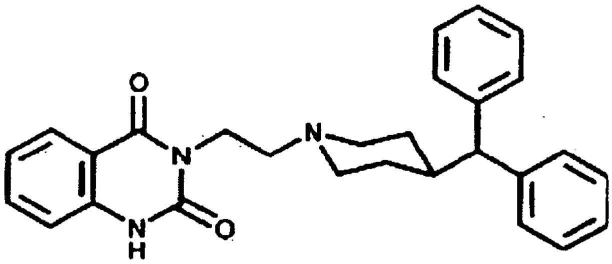

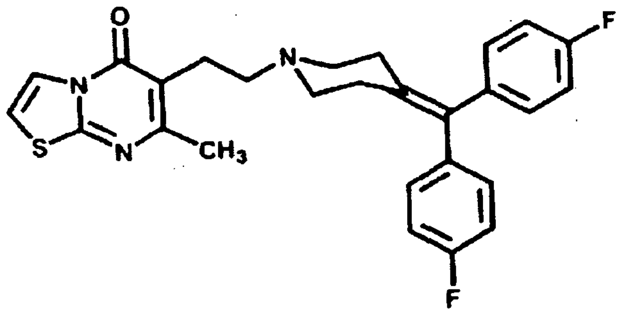

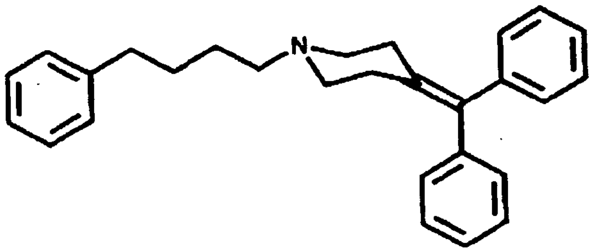

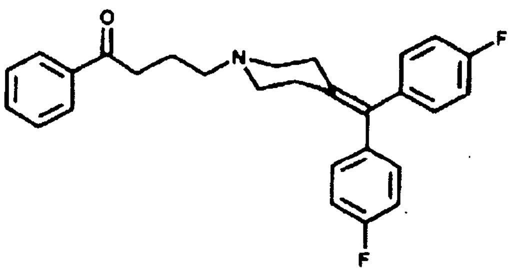

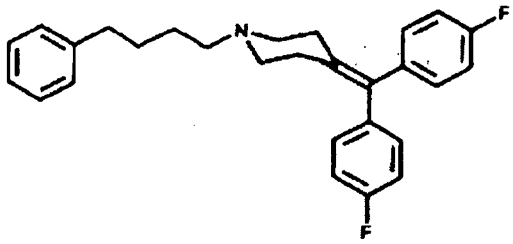

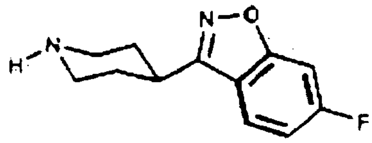

iloperidone:

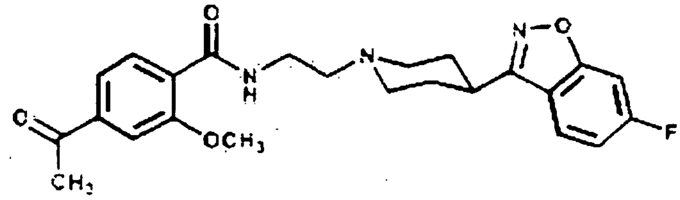

ocaperidone:

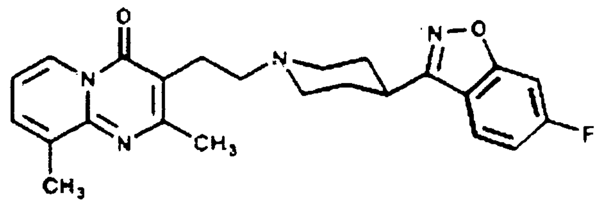

1192U90:

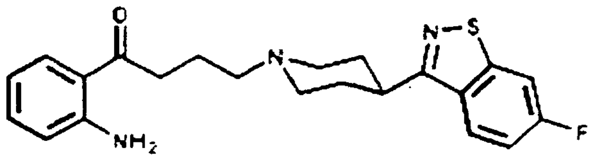

ziprazidone:

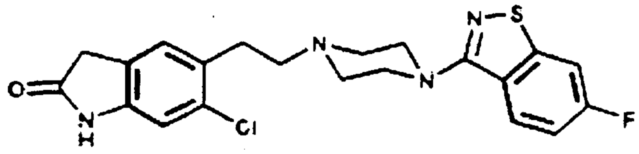

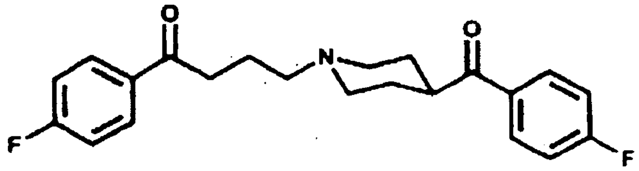

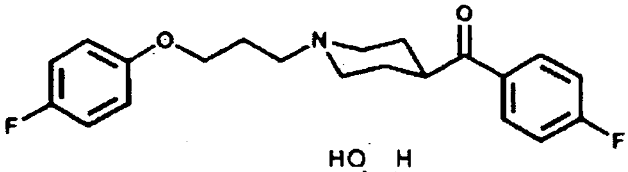

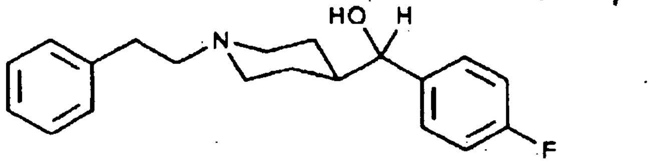

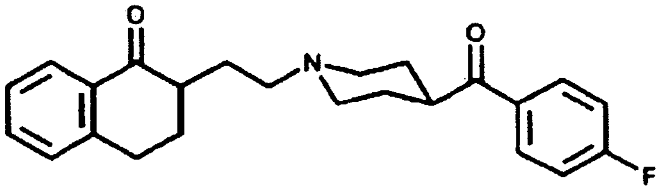

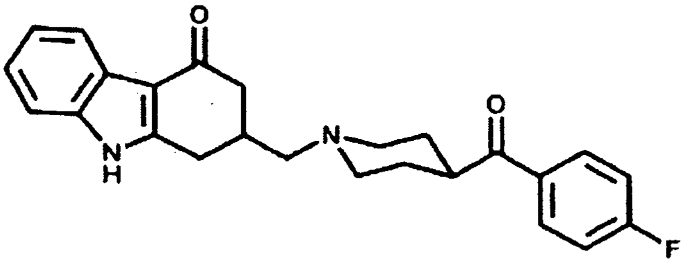

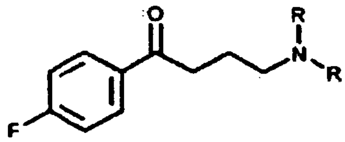

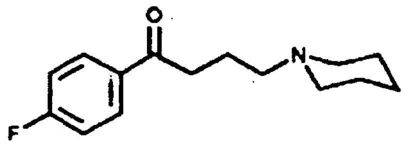

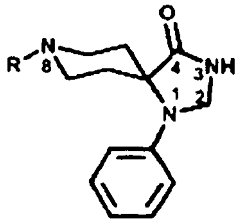

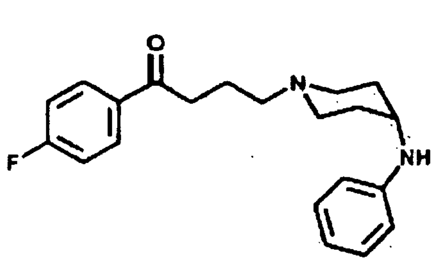

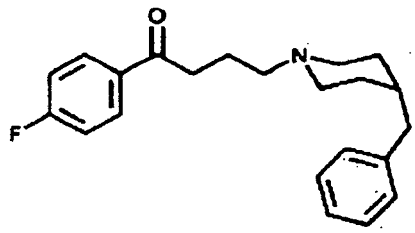

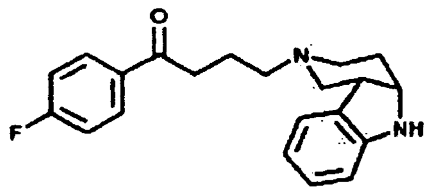

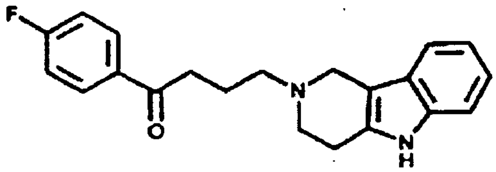

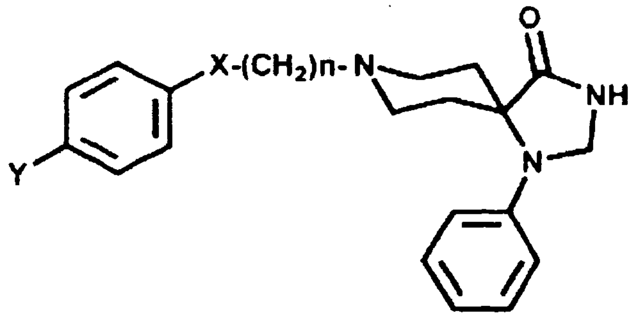

[X=O, Y=H or F, n=3]

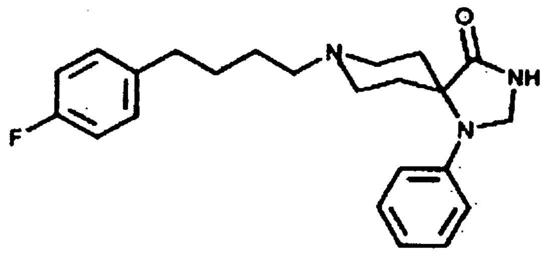

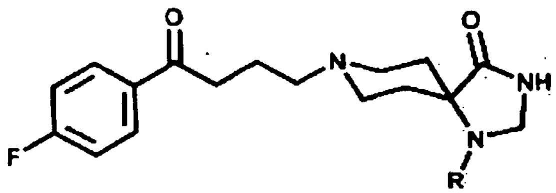

[R=cyclohexyl, isopropyl, or methyl]

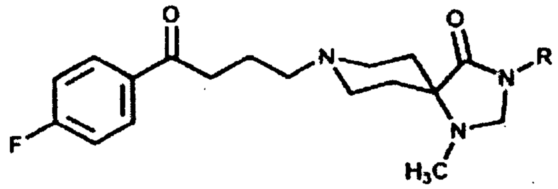

[R=$CH_3$, $CH_2CH_3$, $CH_2CH_2CH_3$, $CH_2CH_2CH_2CH_3$, $CH_2PH$, or isopropyl]

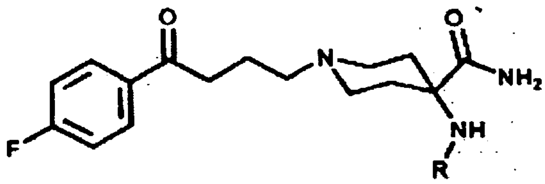

[R=cyclohexyl or methyl]

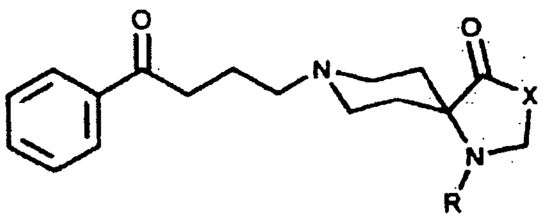

[X=NH, R=Ph; X=$CH_2$, R=Ph; or X=$CH_2$, R=$CH_3$]

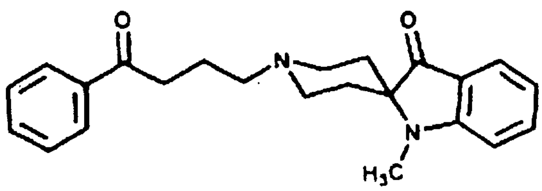

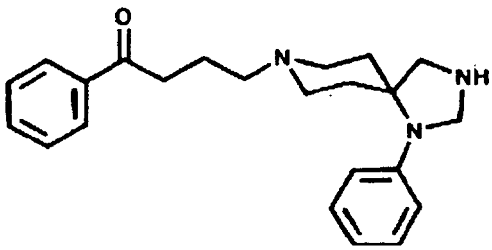

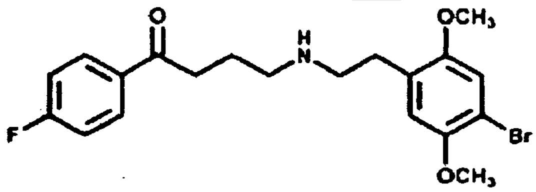

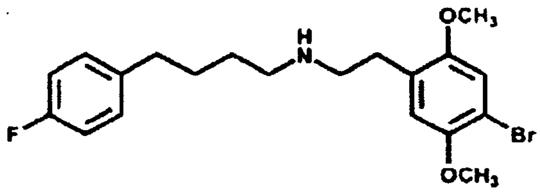

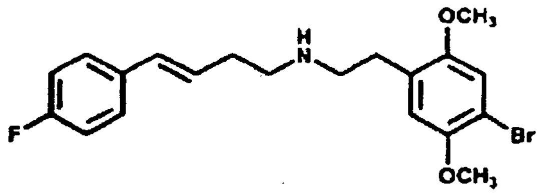

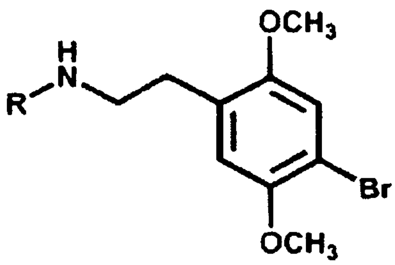

[R=$CH_2CH_2CH_2CH_2$-Ph, -$CH_2CH_2CH_2$-Ph, -$CH_2$-Ph, or $CH_2$-Ph(4-I)]

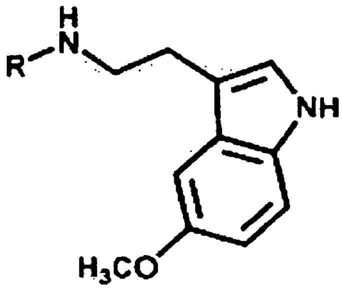

[R=H, $CH_2CH_2CH_2CH_2$-Ph, $CH_2CH_2CH_2$-Ph, $CH_2$-Ph, $CH_2$-PH(4-I), or $CH_2$-Ph(4-Br)]

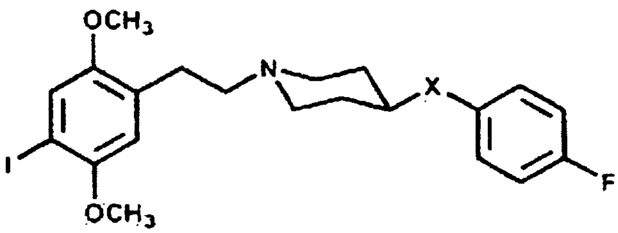

[X=-CO-, -CH(OH)-, or -$CH_2$-]

The antagonistic activity of a 5-HT2A receptor antagonist against the 5-HT2A receptor can be determined easily with the use of a binding assay between a receptor and a ligand, which is well-known in the art. For example, the method of Knight, et al., (A.R. Knight, et al., Naunyn-Schmiedebergs Arch Pharmacol 370:114-123, 2004) can be used. Briefly,

cancer cells (HEK-293, CHO-K1, and HEK-293) introduced with human 5-HT2A, 2B, and 2C receptor genes, respectively are cultured to proliferate. According to a conventional method, cell membrane fractions containing the receptors at a high concentration are prepared from these cells. The maximum binding activity (Bmax) and the dissociation constant (Kd) are determined with the use of $^{125}$ I-DOI(5-HT2A) or $^{3}$H-5-HT(5-HT2B and 5-HT2C) as a labeled compound (ligand) that binds to the receptors. Next, the ability of a drug (5-HT2 antagonist) to inhibit the binding of 0.1 nM of $^{125}$I-labeled DOI(5-HT2A), 5 nM of $^{3}$H-labeled 5-HT (5-HT2B), or 1 nM of $^{3}$H-labeled 5-HT (5-HT2C) to the receptors is tested. An inhibition constant (Ki) is computed from a drug concentration that inhibits 50% of receptor binding (IC50), a radioactive ligand concentration (L), and the Kd value described above. -logKi is pKi. A commercially available software (GraphPad Prism) and a computation formula: Ki=IC50/(1+L/Kd) of Cheng and Prusoff (Biochemical Pharmacol. 22:3099-3108, 1973) are used for the computation of IC50 value and Ki value, respectively.

It is considered that a substance having the binding activity to the 5-HT2A receptor higher than the binding activity to the 5-HT2B and 5-HT2C receptors has an antagonistic activity specific to the 5-HT2A receptor, and has less side effects due to the antagonistic activity on the 5-HT2B and 5-HT2C receptors. For example, it is considered that there is less possibility to aggravate pancreatitis by contraction of blood vessels due to blocking of the 5-HT2B and 5-HT2C receptors.

The pharmaceutical composition of the present invention can be formulated by a method known to those skilled in the art. For example, formulations can be prepared by combining the pharmaceutical component with pharmaceutically acceptable carriers or media, specifically with sterile water, saline, vegetable oil, emulsifier, suspending agent, surface active agent, stabilizer, flavor, excipient, vehicle, antiseptic, binder, and the like and by mixing together in a unit dosage form required for generally accepted pharmaceutical practice.

For oral administration, an extract or a compound or a salt thereof of the present invention can be formulated as tablets, pills, sugar coated tablets, capsules, liquid, gel, syrup, slurry, suspension, or the like by mixing with pharmaceutically acceptable carriers that are well known in the art. Any carriers known in the art may be used, including, for example, an excipient such as lactose, saccharose, sodium chloride, glucose, urea, starch, calcium carbonate, kaolin, crystalline cellulose, and silicic acid; a binder such as water, ethanol, propanol, simple syrup, glucose solution, starch solution, gelatin solution, carboxymethyl cellulose, shellac, methyl cellulose, potassium phosphate, and polyvinylpyrrolidone; a disintegrator such as dried starch, sodium alginate, agar powder, laminaran powder, sodium hydrogencarbonate, calcium carbonate, polyoxyethylenesorbitan fatty acid ester, sodium lauryl sulfate, stearic acid monoglyceride, starch, and lactose; a disintegration inhibitor such as saccharose, stearin, cacao butter, and hydrogenated oil; an absorbefacient such as quarternary ammonium salts and sodium lauryl sulfate; a humectant such as glycerin and starch; an adsorbent such as starch, lactose, kaolin, bentonite, and colloidal silicic acid; a lubricant such as purified talc, stearic acid salt, boric acid powder, and polyethylene glycol, and the like. Further, tablets can be formed with ordinary tablet coating as needed, for example, in sugar coated tablet, gelatin coated tablet, enteric coated tablet, film coated tablet, or double-layer tablet or multilayer tablet.

For parenteral administration, the extract or the compound or the salt thereof of the present invention can be formulated according to the conventional pharmaceutical practice with the use of a pharmaceutically acceptable vehicle well known in the art.

The water-soluble vehicle for injection includes, for example, an isotonic solution containing saline, glucose, and other adjuvants including, for example, D-sorbitol, D-mannose, D-mannitol, and sodium chloride, and may be used in combination with an appropriate solubilizing agent such as alcohol, specifically ethanol and polyalcohol such as propylene glycol and polyethylene glycol, and nonionic surface active agent such as polysorbate 80(TM) and HCO-50

The oily vehicle includes sesame oil and soy oil, and may be used in combination with benzyl benzoate or benzyl alcohol as a solubilizing agent. Further, the oily vehicle may be mixed with a buffer such as phosphate buffer solution and sodium acetate buffer solution, a soothing agent such as procaine hydrochloride, a stabilizer such as benzyl alcohol and phenol, and an antioxidant. The injection thus prepared may be conventionally filled in an appropriate ampule.

An administration route appropriate for the pharmaceutical composition of the present invention includes, but not limited to, oral administration, intrarectal administration, transmucosal administration, enteral administration, intramuscular injection, hypodermic injection, intramedullary injection, intrathecal injection, direct intraventricular injection, intravenous injection, intravitreal injection, intraperitoneal injection, intranasal injection, and intraocular injection. The administration route can be appropriately selected in consideration of the patient's age, conditions, pharmaceutical agents used in combination, and the like.

A dosage of the pharmaceutical composition of the present invention may be selected, for example, within a range from 0.001 mg to 10 mg per kg body weight per dose. Alternatively, a dosage for a patient can be selected from a range of 0.1 to 100 mg per dose; however, the dosage is not necessarily limited to these numerical values. Dosage and administration method can be appropriately selected in consideration of patient's body weight, age, conditions, pharmaceutical agents used in combination, and the like.

In another aspect, the present disclosure provides a method of identifying candidate substances for prophylactic and therapeutic agent for pancreatitis. The method comprises allowing a test substance to contact with cells that express

the 5-HT2A receptor and determining whether the test substance inhibit the binding of the ligand to the 5-HT2A receptor. The binding inhibition assays can be carried out according to the method described above. By these assays, a substance identified as those having 5-HT2A receptor antagonistic activity is regarded as a candidate substance for prophylactic and therapeutic agent for pancreatitis.

The present invention is explained in detail by way of examples below; however, these examples do not limit the scope of the present invention.

Example 1

Receptor subtypes involved in the onset of pancreatitis were examined with the use of antagonists of 5-HT2A receptor, 5-HT2B receptor, and 5-HT2C receptor. A mouse having acute pancreatitis induced by excessive administration of cerulein was used as an animal model. This model is not affected by food consumption, which is different from CDE pancreatitis, and thus it is possible to obtain constant pathologic features and analyze data more accurately.

Method:

An aqueous solution of cerulein (0.05 mg/Kg) was administered to a five-week-old mouse every one hour five times by hypodermic injection. Each 5-HT2 antagonist suspended in a solvent (0.5% methylcellulose in saline) was administered under the dorsal skin 15 minutes before the first administration of cerulein. As the 5-HT2 antagonists, ketanserin (5-HT2A/C antagonist), SB204741 (5-HT2B antagonist), and SB242084 (5-HT2C antagonist) were used in the doses shown in the figure, respectively. Further, only the solvent was administered to the control group (group administered with cerulein alone) under the dorsal skin. One hour after the final administration, blood was collected, and amylase activity and lipase activity in the plasma were determined.

The results are shown in Fig. 1. In the pancreas of the mice administered with cerulein, histology of acute edematous pancreatitis was observed, and amylase and lipase in blood showed about seven to nine times and about 30 to 40 times higher levels compared with those of normal mice, respectively. These results correspond to the experimental facts that have already been published. On the other hand, ketanserin (3.2 mg/Kg) serving as 5-HT2A/C antagonists significantly decreased amylase and lipase in blood of the mice administered with cerulein, whereas SB204741 serving as a 5-HT2B antagonist and SB242084 serving as a 5-HT2C antagonist didn't show any effect.

From these results, it became evident that the 5-HT2A receptor is primary involved in the onset of acute pancreatitis.

Example 2

Effects of various 5-HT2A antagonists on amylase activity and lipase activity in plasma of cerulein-induced pancreatic mice were examined. As the 5-HT2A antagonists, risperidone, spiperone, ketanserin, AMI-193, and MDL11939 were used at the doses shown in the figure and administered to the model mice with cerulein-induced pancreatitis in a way similar to Example 1. Then the amylase activity and the lipase activity in plasma were determined.

The results are shown in Fig. 2. The inhibition rates of amylase activity by each drug (3.2 mg/Kg) were computed. The inhibition rates in descending order of action intensity is: risperidone (52%)>spiperone (41%)>ketanserin (37%) >AMI-193 (17%)>MDL11939 (-4%). Similar order of inhibition rate was observed when examined at other doses and for lipase activity.

Further, the order of these drugs corresponds to the strength of the reorted affinity of each drug for 5-HT2A receptor subtype (Table 1).

[Table 1]

Binding activity of various drugs to 5-HT2 receptor subtypes

| | 5-HT2A pKi±SEM | 5-HT2B pKi±SEM | 5-HT2C pKi±SEM |
|---|---|---|---|
| risperidone | 9.7[a] | | 7.49[a] |
| spiperone | 7.81±0.29 | 5.88±0.12 | 6.22±0.08 |
| ketanserin | 8.09±0.09 | 6.01±0.02 | 7.21±0.12 |
| AMT-193 | 7.65±0.08 | 6.01±0.02 | 5.81±0.16 |
| MDL-11,939 | 7.58±0.12 | 5.48±0.03 | 6.58±0.22 |
| SB242084 | 6.07±0.18 | 6.84±0.28 | 8.15±0.10 |
| SB204741 | <5.00 | 6.90±0.27 | 5.56±0.07 |

(continued)

Binding activity of various drugs to 5-HT2 receptor subtypes

| | 5-HT2A pKi±SEM | 5-HT2B pKi±SEM | 5-HT2C pKi±SEM |
|---|---|---|---|

Knight et al. (2004) Naunyn-Schmiedeber'gs Arch Pharmacol. 370:114-123.
a, Roth et al. (1994) J. Pharmacol. Exp. Ther. 268:1403-1410.

Binding activity of various drugs to 5-HT2 receptor subtypes

| | 5-HT2A pKi±SEM | 5-HT2B pKi±SEM | 5-HT2C pKi±SEM |
|---|---|---|---|
| spiperone | 9.0±0.15 | 5.8±0.05 | <6.00 |
| ketanserin | 8.0±0.11 | 6.2±0.14 | 6.7±0.07 |
| SB204741 | <5.00 | 7.1±0.08 | 5.7±0.04 |

Bonhaus et al. (1995) British Journal of Pharmacology. 115:622-628.

From these results, it became evident that 5-HT2A receptor is involved in the onset of acute pancreatitis and that there is correlation between the affinity of the drugs for 5-HT2A receptor and the inhibition rate of amylase activity.

Example 3

Effects of various 5-HT2 antagonists on amylase activity and lipase activity in plasma of cerulein-induced pancreatic mice were examined. As the 5-HT2 antagonists, metergoline, methysergide, and ritanserin were used at the doses shown in the figure and administered to the model mice with cerulein-induced pancreatitis in a way similar to Example 1. Then the amylase activity and the lipase activity in plasma were determined. As shown in Table 2, these 5-HT2 antagonists have the binding activity to the 5-HT2A receptor higher than that of ketanserin and as much as that of spiperone, and also have high binding activity to the 5-HT2B receptor and the 5-HT2C receptor.

[Table 2]

Binding activity of various drugs to 5-HT2 receptor subtypes

| | 5-HT2A pKi±SEM | 5-HT2B pKi±SEM | 5-HT2C pKi±SEM |
|---|---|---|---|
| Methysergide | 8.40±0.16 | 9.44±0.05 | 8.60±0.05 |
| Ritanserin | 8.34±0.09 | 8.67±0.09 | 8.18±0.15 |
| Metergoline | 8.64±0.07 | 8.75±0.08 | 8.75±0.11 |

Knight et al. (2004) Naunyn-Schmiedeber'gs Arch Pharmacol. 370:114-123.

The results are shown in Fig. 3. When the inhibition rates of amylase by each drug (3.2 mg/kg) were computed, the inhibition rates by metergoline and spiperone were 30% and 15%, respectively, and no inhibition was shown by methysergide. In any case, the inhibition rates were lower than those by ketanserin and spiperone. In other words, it became evident that broad-spectrum substances having the binding activity to the 5-HT2A receptor, the 5-HT2B receptor, and the 5-HT2C receptor have a weak effect on suppression of pancreatitis compared with substances having the binding activity to the 5-HT2A receptor higher than that to the 5-HT2B receptor and the 5-HT2C receptor.

From the above results, it was found that 5-HT released by pancreatic juice secretion hormone such as cerulein stimulates the 5-HT2A receptor, which causes the onset of pancreatitis. On the other hand, it was conceived that the released 5-HT also stimulates 5-HT2B receptor and 5-HT2C receptor at the same time, and this stimulation increases blood flow, thereby working to suppress the onset of pancreatitis. These findings suggests that antagonists specific to 5-HT2A receptor subtype among 5-HT2 receptor antagonists are useful for prevention and treatment of pancreatitis.

INDUSTRIAL APPLICABILITY

The present invention is useful in prophylaxis and treatment of pancreatitis.

## Claims

1. A pharmaceutical composition comprising a 5-HT2A receptor antagonist as an effective component, for use in prophylaxis and treatment of pancreatitis, wherein the binding activity (pKi) of the 5-HT2A receptor antagonist to a 5-HT2A receptor is higher at least by 1.0 than the binding activity to a 5-HT2B receptor and is higher at least by 1.0 than the binding activity to a 5-HT2C receptor,
and wherein the 5-HT2A receptor antagonist is selected from the group consisting of risperidone, ziprasidone, paliperidone, iloperidone, quetiapine, nefazodone, perospirone, aripiprazole, zotepine, mirtazapine, sertindole, asenapine, blonanserin, spiperone, amperozide, and chlorpromazine.

2. The pharmaceutical composition according to claim 1 for use according to claim 1, wherein the binding activity (pKi) of the 5-HT2A receptor antagonist to the 5-HT2A receptor is at least 7.0.

3. The pharmaceutical composition according to claim 1 for use according to claim 1, wherein the binding activity (pKi) of the 5-HT2A receptor antagonist to the 5-HT2A receptor is at least 8.0.

4. The pharmaceutical composition according to claim 1 for use according to claim 1, wherein the binding activity (pKi) of the 5-HT2A receptor antagonist to the 5-HT2A receptor is at least 9.0.

## Patentansprüche

1. Pharmazeutische Zusammensetzung umfassend einen 5-HT2A Rezeptor Antagonisten als ein wirksamer Bestandteil zur Verwendung in der Prophylaxe und Behandlung von Pankreatitis, wobei die Bindungsaktivität (pKi) des 5-HT2A Rezeptor Antagonisten zu einem 5-HT2A Rezeptor mindestens um 1,0 höher ist als die Bindungsaktivität zu einem 5-HT2B Rezeptor und mindestens um 1,0 höher ist als die Bindungsaktivität zu einem 5-HT2C Rezeptor, und wobei der 5-HT2A Rezeptor Antagonist ausgewählt ist aus der Gruppe bestehend aus Risperidon, Ziprasidon, Paliperidon, Iloperidon, Quetiapin, Nefazodon, Perospiron, Aripiprazol, Zotepin, Mirtazapin, Sertindol, Asenapin, Blonanserin, Spiperon, Amperozid und Chlorpromazin.

2. Die pharmazeutische Zusammensetzung gemäß Anspruch 1 zur Verwendung gemäß Anspruch 1, wobei die Bindungsaktivität (pKi) des 5-HT2A Rezeptor Antagonisten zum 5-HT2A Rezeptor mindestens 7,0 ist.

3. Die pharmazeutische Zusammensetzung gemäß Anspruch 1 zur Verwendung gemäß Anspruch 1, wobei die Bindungsaktivität (pKi) des 5-HT2A Rezeptor Antagonisten zum 5-HT2A Rezeptor mindestens 8,0 ist.

4. Die pharmazeutische Zusammensetzung gemäß Anspruch 1 zur Verwendung gemäß Anspruch 1, wobei die Bindungsaktivität (pKi) des 5-HT2A Rezeptor Antagonisten zum 5-HT2A Rezeptor mindestens 9,0 ist.

## Revendications

1. Une composition pharmaceutique comprenant un antagoniste du récepteur 5-HT2A en tant que principe actif, pour une utilisation pour la prophylaxie et le traitement de la pancréatite, dans laquelle l'activité de liaison (pKi) de l'antagoniste du récepteur 5-HT2A vis-à-vis d'un récepteur 5-HT2A est supérieure d'au moins 1,0 à l'activité de liaison vis-à-vis d'un récepteur 5-HT2B, et est supérieure d'au moins 1,0 à l'activité de liaison vis-à-vis d'un récepteur 5-HT2C et dans laquelle l'antagoniste du récepteur 5-HT2A est choisi dans le groupe constitué par la rispéridone, la ziprasidone, la palipéridone, ilopéridone, la quétiapine, la néfazodone, la perospirone, l'aripiprazole, la zotépine, la mirtazapine, le sertindole, l'asénapine, la blonanserine, la spipérone, l'ampérozide, et la chlorpromazine..

2. La composition pharmaceutique selon la revendication 1, pour une utilisation selon la revendication 1, dans laquelle l'activité de liaison (pKi) de l'antagoniste du récepteur 5-HT2A vis-à-vis du récepteur 5-HT2A est d'au moins 7,0.

3. La composition pharmaceutique selon la revendication 1, pour une utilisation selon la revendication 1, dans laquelle l'activité de liaison pKi) de l'antagoniste du récepteur 5-HT2A vis-à-vis du récepteur 5-HT2A est d'au moins 8,0.

4. La composition pharmaceutique selon la revendication 1, pour une utilisation selon la revendication 1, dans laquelle l'activité de liaison (pKi) de l'antagoniste du récepteur 5-HT2A vis-à-vis du récepteur 5-HT2A est d'au moins 9,0.

FIG. 1

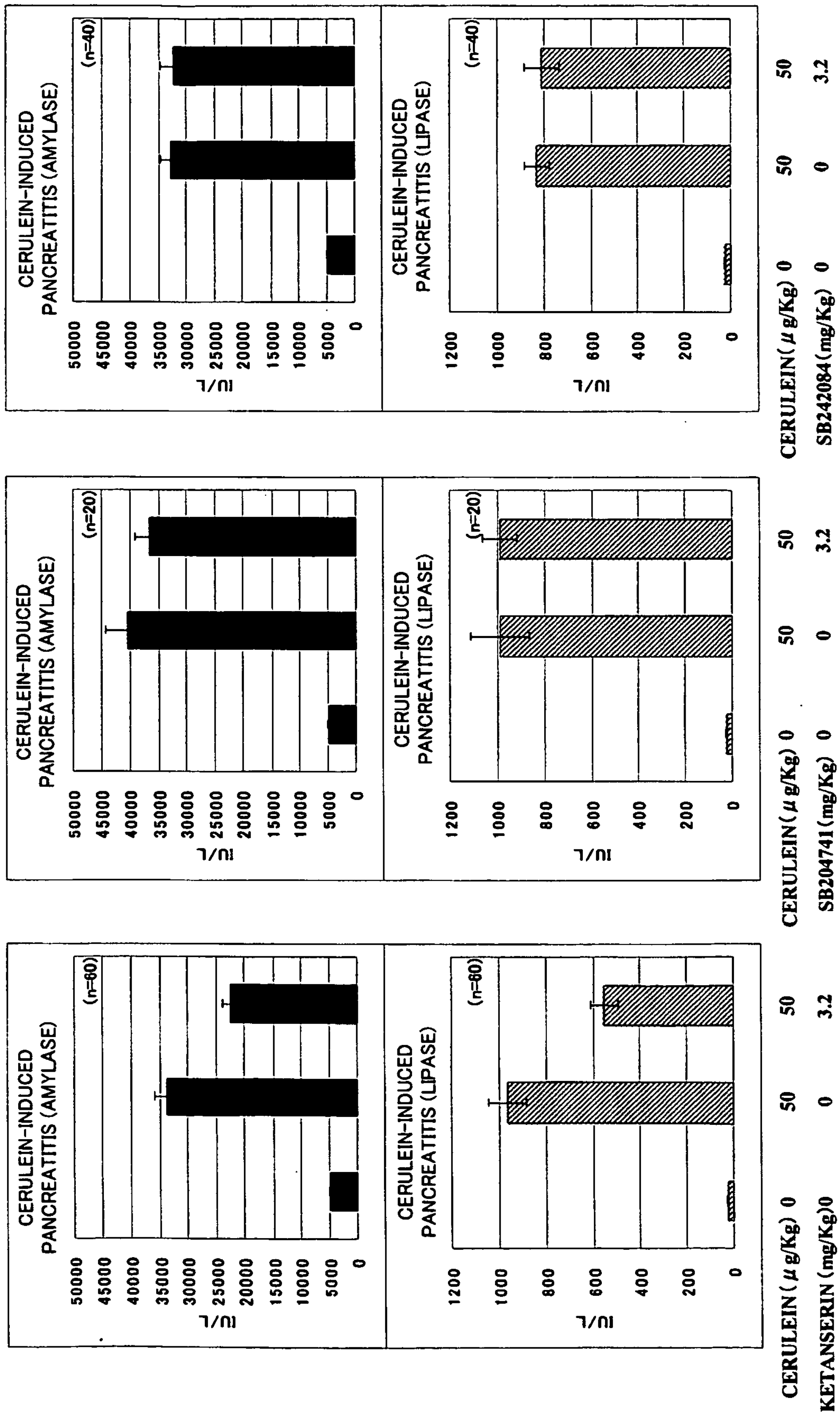
CERULEIN-INDUCED PANCREATITIS (AMYLASE)
CERULEIN-INDUCED PANCREATITIS (LIPASE)
IU/L
(n=80)
(n=20)
(n=40)
CERULEIN (μg/Kg) 0 50 50
KETANSERIN (mg/Kg) 0 0 3.2
SB204741 (mg/Kg) 0 0 3.2
SB242084 (mg/Kg) 0 0 3.2

FIG. 2

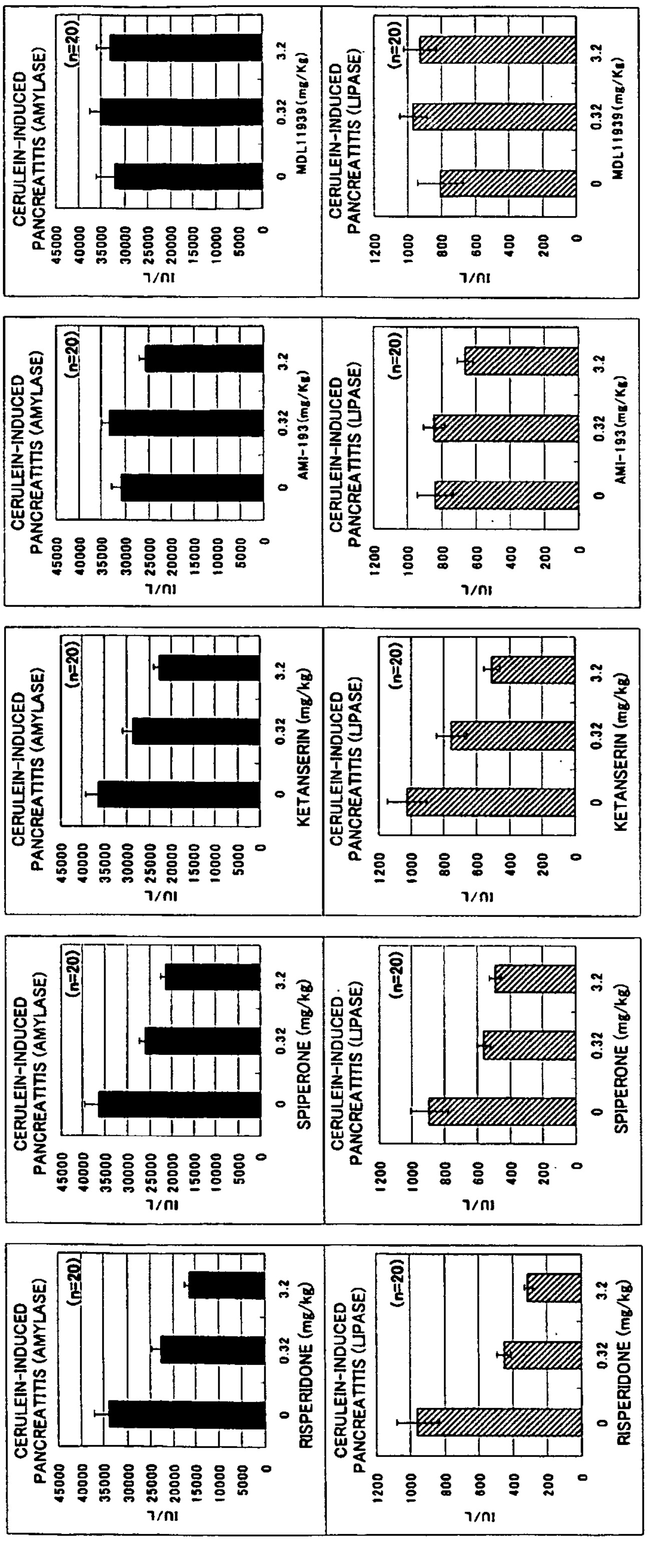
CERULEIN-INDUCED PANCREATITIS (AMYLASE)
CERULEIN-INDUCED PANCREATITIS (LIPASE)
(n=20)
IU/L
0 0.32 3.2
MDL11939 (mg/Kg)
AMI-193 (mg/Kg)
KETANSERIN (mg/kg)
SPIPERONE (mg/kg)
RISPERIDONE (mg/kg)

FIG. 3

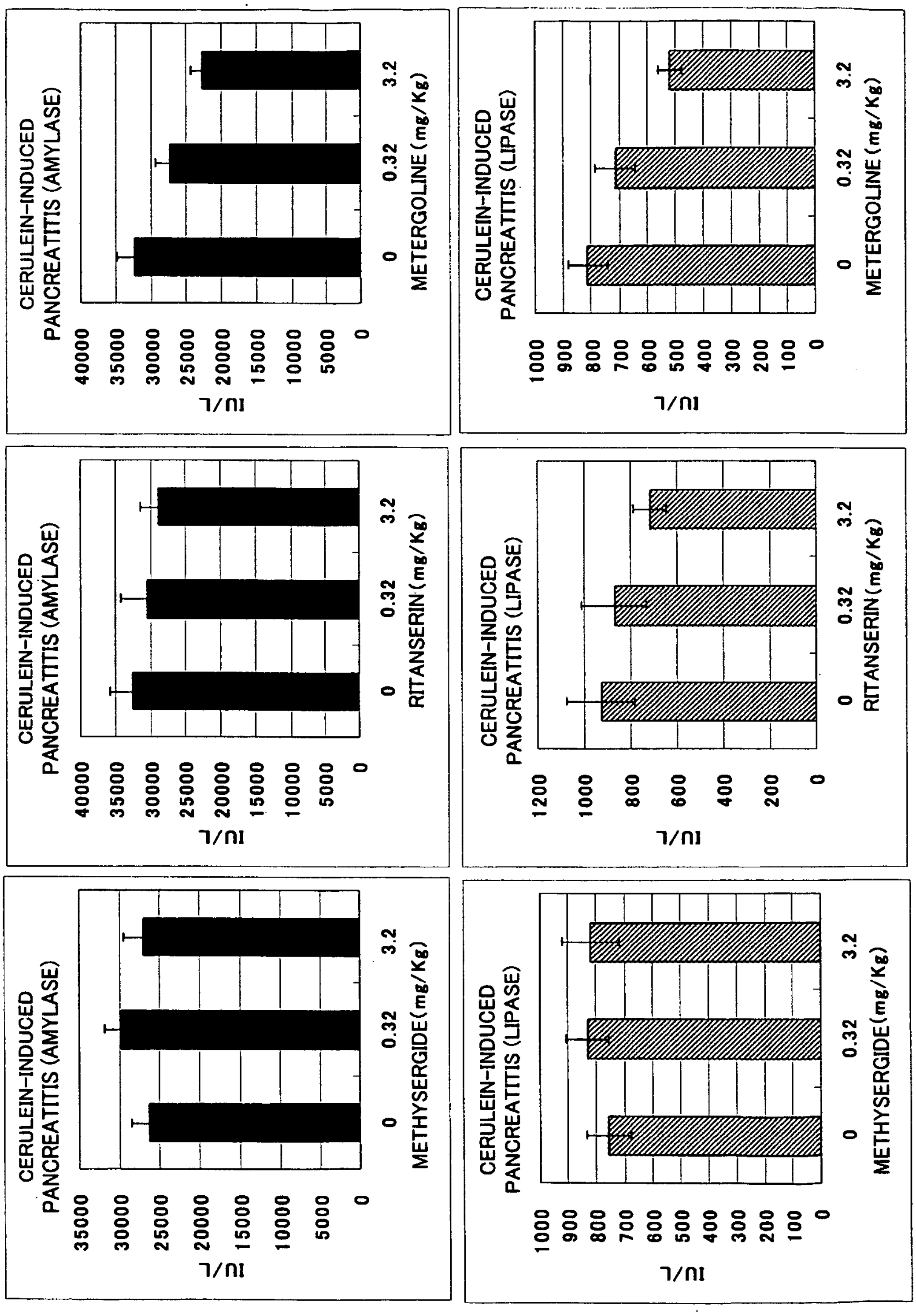
CERULEIN-INDUCED PANCREATITIS (AMYLASE)
IU/L
40000
35000
30000
25000
20000
15000
10000
5000
0
0
0.32
3.2
METERGOLINE (mg/Kg)
CERULEIN-INDUCED PANCREATITIS (LIPASE)
IU/L
1000
900
800
700
600
500
400
300
200
100
0
0
0.32
3.2
METERGOLINE (mg/Kg)
CERULEIN-INDUCED PANCREATITIS (AMYLASE)
IU/L
40000
35000
30000
25000
20000
15000
10000
5000
0
0
0.32
3.2
RITANSERIN (mg/Kg)
CERULEIN-INDUCED PANCREATITIS (LIPASE)
IU/L
1200
1000
800
600
400
200
0
0
0.32
3.2
RITANSERIN (mg/Kg)
CERULEIN-INDUCED PANCREATITIS (AMYLASE)
IU/L
35000
30000
25000
20000
15000
10000
5000
0
0
0.32
3.2
METHYSERGIDE (mg/Kg)
CERULEIN-INDUCED PANCREATITIS (LIPASE)
IU/L
1000
900
800
700
600
500
400
300
200
100
0
0
0.32
3.2
METHYSERGIDE (mg/Kg)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10212232 A
- JP 2000063270 A
- JP 10147528 A


### Non-patent literature cited in the description


- **YOSHINO et al.** *J. Pharmacol. Exp. Ther.,* 1997, vol. 283 (3), 1495-1502
- **KOLLER et al.** *Pharmacotherapy,* 2003, vol. 23 (9), 1123-1130
- **OGUCHI et al.** *Life Sciences,* vol. 50, 733-737
- **BONHAUS et al.** *Br.J.Pharmacology,* vol. 115, 622-628
- **GLENNON et al.** *Curr Top Med Chem,* 2002, vol. 2, 539-558
- **A.R. KNIGHT et al.** *Naunyn-Schmiedebergs Arch Pharmacol,* 2004, vol. 370, 114-123
- **CHENG ; PRUSOFF.** *Biochemical Pharmacol.,* 1973, vol. 22, 3099-3108
- **KNIGHT et al.** *Naunyn-Schmiedeber'gs Arch Pharmacol.,* 2004, vol. 370, 114-123
- **ROTH et al.** *J. Pharmacol. Exp. Ther.,* 1994, vol. 268, 1403-1410
- **BONHAUS et al.** *British Journal of Pharmacology,* 1995, vol. 115, 622-628